# EUROPÄISCHE PATENTSCHRIFT

(11) **EP 1 487 959 B1**
(45) Veröffentlichungstag und Bekanntmachung des Hinweises auf die Patenterteilung: **14.10.2009**
(21) Anmeldenummer: 03744763.8
(22) Anmeldetag: 22.03.2003
(51) Int. Cl.: C12G 1/028, G01N 33/14

(54) **VERFAHREN ZUR GESTEUERTEN GARFUHRUNG VON GETRANKEN MIT HILFE VON SENSOREN BZW. AUTOMATISIERTER ANALYSEMETHODEN**
METHOD FOR THE CONTROLLED FERMENTATION OF DRINKS BY MEANS OF SENSORS AND AUTOMATED ANALYTICAL METHODS
PROCEDE DE FERMENTATION REGULEE DE BOISSONS A L'AIDE DE CAPTEURS ET METHODES D'ANALYSE AUTOMATISEES

(30) Priorität: 22.03.2002 DE 10212773
(43) Veröffentlichungstag der Anmeldung: 22.12.2004
(73) Patentinhaber: Griebl, Matthias, 77855 Achern (DE); Berres, Markus, 54539 Ürzig Mosel (DE)
(72) Erfinder: Griebl, Matthias, 77855 Achern (DE); Berres, Markus, 54539 Ürzig Mosel (DE)
(74) Vertreter: Fuchs
(86) Internationale Anmeldenummer: PCT/DE2003/000957
(87) Internationale Veröffentlichungsnummer: WO 2003/079826

(56) Entgegenhaltungen:
- EP-A- 0 271 380
- DE-A- 2 323 416
- DE-A- 3 434 170
- DE-A- 4 129 174
- DE-C- 917 122

## Beschreibung

Die Erfindung betrifft ein Verfahren zur Herstellung von alkoholischen und/oder milchsäurehaltigen Nahrungsmitteln, insbesonders von Getränken mittels Mikroorganismen und/oder mittels daraus gewonnenen Fermenten in einer wässrigen Fermentationsbrühe, sowie eine Vorrichtung zur Durchführung des Verfahrens.

Bei der alkoholischen Gärung entsteht beim Abbau von Zukkern Wärme. Diese Wärme erhöht nicht nur die Temperatur der Fermentationsbrühe bzw. Maische, sondern führt auch zu einer Verstärkung der Gärung und damit auch zu einem schnelleren Abbau des Zuckers. Es ist jedoch bekannt, dass eine zu schnelle bzw. stürmische Gärung zu einer Erhöhung an unerwünschten Nebenprodukten führt und dass bei einer erhöhten Gärtemperatur die für den Geschmack besonders wichtigen Duft- und Bukettstoffe ausgetrieben werden, so dass ein auf diese Weise erzeugtes Produkt, wie beispielsweise Wein, von geringerer sensorischer Qualität ist. Üblicherweise wird dann nach der Gärung versucht solche Gärfehler durch eine nachträgliche Behandlung des Weines wieder auszugleichen, was jedoch nur unzulänglich gelingt.

Eine Nachbehandlung von Weinen ist jedoch insbesondere bei anspruchsvollen Verbrauchern unerwünscht. Daher wird allgemein angestrebt, ein möglichst naturnahes unverfälschtes Produkt zu erzeugen. Dies ist beispielsweise bei der Weinherstellung nur dadurch zu erreichen, dass eine möglichst fehlerfreie Gärung durchgeführt wird, so dass der Wein so wenig wie möglich nachbehandelt werden muss. Dies bedeutet, dass der Verbraucher ein Produkt erwartet, welches keine sensorischen, d. h. geschmacklichen optischen und/oder geruchlichen Fehler aufweist.

Die Steuerung der Gärung bzw. der Fermentation wird bereits seit vielen Jahren mit verschiedenen Mitteln durchgeführt, wobei im Wesentlichen allein die Gärtemperatur geregelt wird. Die Gärtemperatur erhöht nämlich automatisch die Stoffwechselaktivität der gärenden Mikroorganismen, insbesondere der Hefen, wobei die freiwerdende Gärwärme sowie die starke Stoffwechselaktivität zur Bildung von unerwünschten Geschmacksausprägungen führt, z.B. sogenannten off-flavor-Verbindungen, wie flüchtige schwefelhaltige Verbindungen etc. Dies soll durch eine temperaturkontrollierte Gärung verhindert werden.

Prinzipiell führt eine Vergärung bei tieferen Temperaturen, z.B. bei der sogenannten Kaltvergärung, zur Bildung von gewünschten Aromen, wie beispielsweise fruchtigen bonbonartigen Geschmackstönen, wobei eine Korrelation zwischen Gärtemperatur, Gärgeschwindigkeit und Aromaintensität besteht. Es hat sich jedoch gezeigt, dass eine Kaltvergärung sehr leicht dazu führt, dass bei größeren Tankvolumen wie sie in Großkellereien eingesetzt werden, die entstehende Gärwärme nicht mehr im ausreichenden Maße abgeführt werden kann, wenn bereits eine verstärkte Gärgeschwindigkeit eingesetzt hat, so dass eine bereits entstandene starke Gärung nur schwer oder nur durch rigide Kühlung wieder unter Kontrolle gebracht werden kann. Andererseits kann es vorkommen, dass bei zu tiefer Temperatur der Gärprozess ganz zum Erliegen kommt oder nur mit besonderen Maßnahmen wieder in Gang gebracht werden kann, wie Filtrieren und Klären. Ein einfaches Erwärmen reicht dabei oft nicht aus, so dass erhöhte Kosten und weitere Risiken bestehen. Es werden daher bereits Temperatursensoren zusammen mit einer entsprechenden Regeltechnik eingesetzt, mit welcher die jeweiligen Gärbehälter auf eine ideale Gärtemperatur eingestellt werden. Dabei geben die Temperatursensoren, die an verschiedenen Stellen innerhalb eines Gärtankes angeordnet sind, Messwerte an eine Regelungselektronik ab, die wiederum mittels Kühlelementen, wie Kühltaschen oder Kühlmänteln, die Temperatur des Tankinhaltes regelt. Bei größeren Tankinhalten muss jedoch in der Regel bereits so stark gekühlt werden, dass es zu Teilunterkühlungen des Gärgutes führt, was wiederum einen fatalen Einfluss auf die Gesamtfermentation hat.

Eine wie zuvor beschriebene temperaturkontrollierte Gärung wird bereits zur Herstellung einer Vielzahl von Weinen eingesetzt. So ist es insbesonders für Weißwein und in manchen Fällen auch für Rotwein bekannt eine temperierte Gärung einzusetzen. Es ist aber auch bekannt die zu gärende Rotweinmaische während der Fermentation zu kühlen. Der Most wird vom Kellermeister bei einer festgelegten Temperatur vergoren, die meist auf Erfahrungswerte der vergangenen Jahre zurückzuführen ist. Dabei wird zuerst einmal nicht berücksichtigt, dass unterschiedliche Jahrgänge unterschiedliche Ansprüche an die Gärtemperatur haben. Wird später dann erkannt, dass die Jahrgänge doch nicht vergleichbar sind, dann muss oftmals eine Änderung der Temperatur von Hand erfolgen. Gekühlte Verfahren haben den Vorteil, dass die besonders zum Aroma beitragenden flüchtigen Bukettstoffe weniger stark entweichen. Auch entweicht durch die niedrigere Temperatur weniger Alkohol, so dass der so erhaltene Wein alkoholreicher ist.

In der DE-C-917 122 wird eine Anordnung zur Steuerung der Gärung von Weiß- und Rotwein beschrieben. Dabei werden in einem Drucktank, der mit üblichen Messgeräten ausgerüstet ist, die Absolutwerte physikalischer Parameter wie Temperatur, Druck und auch Öchsle-Grade bestimmt, und beim Überschreiten vorgegebener absoluter Sollwerte wird der Tank mittels einer Berieselungsanlage solange gekühlt, bis die Werte wieder innerhalb eines vorgegebenen Sollbereiches liegen.

Die DE-A-43 05 167 beschreibt ein Verfahren zur vollautomatischen Gärprozessregelung bei der Herstellung alkoholischer Getränke, bei der der Alkoholgehalt über einen Gärabgasstrom erfasst wird und durch Temperatur- und Druckänderungen entsprechend geregelt wird.

In der DE-C-195 47 473 wird ein Verfahren zur Konzentrationsermittlung von Sekundärstoffwechselprodukten in einem chargenbetriebenen Fermentationsprozess von Bier beschrieben. Dabei werden physikalische und chemische Parameter des Gutes anhand der Temperatur, des pH-Wertes und der Dichte des Fermentationsgutes ermittelt und einem neuronalen Netz zugeführt, das einen Schätzwert für die Konzentration der Sekundärstoffwechselprodukte ausgibt. Das hierbei für die Bierfermentation wesentliche Stoffwechselprodukt ist die Diacetylkonzentration. Diese wird jedoch nicht selbst messtechnisch bestimmt, sondern lediglich anhand leicht messbarer Stoffgrößen, wie Temperatur, Druck etc. und der Verlauf dieser Größen seit Prozessbeginn durch Vergleichswerte errechnet. US-A-5,204,262 beschreibt einen Alkoholsensor zur computergesteuerten Fermentationskontrolle, und zwar mittels einem SnO₂-Sensor.

Es hat sich jedoch gezeigt, dass diese Fermentationskontrollen nicht sicherstellen, dass hierdurch ein befriedigendes Geschmacksergebnis sowie eine Kontrolle/Überwachung und Steuerung der Fermentation erreicht wird. Dies liegt vor allem daran, dass bei der Fermentation nicht nur Hefe eine wichtige Rolle spielt, sondern dass mit dem Fermentations- bzw. Gärgut auch eine Vielzahl von anderen Mikroorganismen eingetragen werden, die ebenfalls während der Fermentation aktiv werden können und die andere zum Teil gewünschte wie auch unerwünschte Geschmacksnoten erzeugen. Ein wesentlicher Mikroorganismus hierbei sind beispielsweise Milchsäurebakterien, die in der Lage sind, Äpfelsäure abzubauen und damit den Säuregehalt bzw. die geschmacklich wahrnehmbare Säure im Wein zu reduzieren, z. B. durch Säuren mit höheren pK-Werten, Erhöhung des pH-Wertes. Dies wird auch als biologischer Säureabbau (BSA) bezeichnet. Darüber hinaus wurde gefunden, dass neben geringen Restzuckergehalten auch die vollständig abgebaute Äpfelsäure ggf. vorliegenden weinschädigenden Mikroorganismen nahezu jegliche Wachstums- und Stoffwechselmöglichkeiten entzieht, wodurch der Grad der Schwefelung reduziert werden kann. Dabei führt ein gezielter biologischer Säureabbau (BSA) zu besonders sensorisch ausgewogenen Säurewerten, wobei selbst in extrem schlechten Jahrgängen durch einen biologisch gesteuerten Säureabbau noch eine Verbesserung des Endproduktes erreicht werden kann. Selbstverständlich trägt auch in guten Jahrgängen der BSA zur Harmonisierung des Produktes bei. Auch Nebenprodukte des BSA wie die butterigen Töne von Diacetyl beeinflussen den Gesamteindruck des Geschmacks. Es hat sich nun gezeigt, dass es von Vorteil ist, wenn die alkoholische Gärung und beispielsweise die malolactische Gärung nicht gemeinsam, sondern nacheinander ablaufen, da bei gemeinsamem gleichzeitigem Ablauf andere Nebenprodukte erzeugt werden als wenn diese verschiedenen Gärungen nacheinander durchgeführt werden. Dabei hat es sich als besonders vorteilhaft erwiesen wenn die malolactische Gärung erst nach Beendigung der alkoholischen Gärung durchgeführt wird. Dies gilt auch für viele andere Fermentationsprozesse.

Die Erfindung hat nun zum Ziel, ein Verfahren bereitzustellen, bei welchem die Fermentation besser gelenkt bzw. gesteuert werden kann. Die Erfindung hat auch zum Ziel, auch mit schlechteren Ausgangswerten eines zu fermentierenden Lebensmittels oder Getränkes, wie beispielsweise schlechteren Weinjahrgängen, noch zufriedenstellende Produkte zu erzeugen. Dabei soll allein durch eine rechtzeitige Temperaturänderung unerwünschte Nebenreaktionen verringert, und sofern möglich, vollständig gestoppt oder verhindert werden.

Dieses Ziel wird nun mittels dem in den Ansprüchen definierten Verfahren und der Vorrichtung erreicht.

Erfindungsgemäß wurde nämlich gefunden, dass durch eine Änderung der Konzentration spezieller Fermentationssubstrate und/oder der Fermentationsprodukte pro Zeit bereits frühzeitig das Einsetzen von unerwünschten Reaktionen erkannt werden kann, und zwar lange bevor dies durch eine Temperaturänderung der Fermentationsbrühe bzw. Maische und/oder Most merkbar wird. Damit ist es möglich bereits rechtzeitig die unerwünschte Reaktion durch eine Temperaturänderung zu verringern oder möglichst ganz zu stoppen. Auf diese Weise ist es beispielsweise möglich bei auftretenden Fehlfermentierungen die Temperatur sofort so weit zu ändern, bis nur noch oder fast nur noch die gewünschte Reaktion abläuft bzw. wenn nötig die Temperatur soweit zu senken, bis alle Stoffwechselprozesse zum Erliegen kommen um so eine weitere Fehlfermentation zu vermeiden. Vorzugsweise wird dies durch eine Termperaturabsenkung erreicht, wie beispielsweise bei der alkoholischen Gärung. Erst nach Beendigung der alkoholischen Gärung kann dann durch entsprechende Temperaturerhöhung beispielsweise eine malolactische Gärung durchgeführt werden.

Die im erfindungsgemäßen Verfahren zur Prozesssteuerung verwendeten Parameter umfassen die Temperaturänderung pro Zeit und mindestens einen, vorzugsweise mindestens zwei Parameter, die ausgewählt sind aus Abnahme der Gesamtzuckerkonzentration pro Zeit, Verhältnis von Glucose- zu Fructosekonzentration, Abnahme von Malatkonzentration und/oder Zunahme von Lactatkonzentration pro Zeit. Vorzugsweise werden jedoch mindestens 3, insbesonders mindestens 4 Parameter verwendet. In einer besonders bevorzugten Ausführungsform werden alle Parameter zur Steuerung herangezogen. Überschreitet ein Parameter einen Sollgrenzwert, d. h. fällt er aus einem gewählten Bereich heraus, dann wird die Temperatur der Fermentationsbrühe so weit und so lange geändert, bis dieser Parameter wieder seinen Sollwert erreicht hat, und/oder eine dieses Überschreiten des Grenzwertes auslösende unerwünschte Stoffwechselreaktion zum Erliegen kommt bzw. zumindest verringert oder minimiert wird.

Mittels dem erfindungsgemäßen Verfahren ist es auch möglich, den Ablauf einzelner Fermentationsarten, wie beispielsweise die alkoholische Gärung und/oder die malolactische Gärung jeweils separat zu steuern und dadurch andere unerwünschte chemische Prozesse zu verhindern. Entsprechende Temperaturänderungen ermöglichen auch ein beliebiges Produkt auf einen gewünschten Restwert einzustellen, wie beispielsweise den Gehalt an Restzucker, den Säuregehalt und/oder den Gehalt an Acetat so gering wie möglich zu halten.

Das erfindungsgemäße Verfahren wird vorzugsweise derart durchgeführt, dass die Änderung der Temperatur der Fermentationsbrühe maximal 5°C/Stunde, vorzugsweise maximal 4°C/Stunde, insbesondere maximal 3°C/Stunde bzw. maximal 2°C/ Stunde beträgt. Je geringer die Temperaturänderung pro Zeit ist, um so besser können sich die Mikroorganismen auf die neue Temperatur einstellen. Dabei beträgt die Temperatur der Fermentationsbrühe vorzugsweise 3-35°C. Sie ist auch abhängig von den verwendeten Mikroorganismen und der angestrebten Geschmacksausprägung, in vielen Fällen beträgt sie zwischen 12°C und 22°C, wobei bei BSA die Temperatur > 20°C, besser mindestens gleich 22°C beträgt. Das Optimum bei der alkoholischen Gärung ist dann erreicht, wenn die Gärung ruhig und möglichst stetig abläuft, also weder starke Zu- noch Abnahmen der Gärintensität zu verzeichnen sind. Auch die Temperatur der Kühlvorrichtung sollte diesen Bereich um nicht mehr als 1°C über- bzw. unterschreiten, damit im Bereich der Kühlvorrichtung keine lokale Unterkühlung bzw. Überhitzung erfolgt. Vorzugsweise liegt die Temperatur des Heiz- und/oder Kühlmittels jedoch innerhalb dieses Bereiches. Vorzugsweise sollte sich die Temperatur der Heiz- und/oder Kühlvorrichtung nicht mehr als 10°C, vorzugsweise nicht mehr als 5°C und insbesonders nicht mehr als 4° C bzw. 3°C von der Temperatur der Fermentationsbrühe unterscheiden und zwar sowohl beim Abkühlen als auch Erwärmen. Bei richtiger Dimensionierung der Kühlleistung kann jedoch die Kühlvorrichtung oder das Kühlmedium generell, d. h. unabhängig von der Temperatur der Fermentationsbrühe, eine Temperatur von etwa 4°C aufweisen. Darüber hinaus hat es sich erfindungsgemäß gezeigt, dass eine Änderung der Fermentationstemperatur beispielsweise um 1°C sich bei niedrigen Temperaturen, wie z. B. bei 8°C, anders auswirkt als die gleiche Änderung bei einer erhöhten Temperatur, wie beispielsweise bei 15°C oder 20°C. Derartige Wirkungen sind jedoch vom Fachmann leicht mittels einer Eichkurve zu ermitteln, so dass diese bei der Berechnung der Kühl- bzw. Heizleistung mit einbezogen werden können.

Erfindungsgemäß hat es sich gezeigt, dass die Abnahme des Gesamtzuckergehaltes pro Stunde nicht weniger als 0,05 g/l und nicht mehr als 2,2 g/l betragen sollte. Vorzugsweise liegt sie zwischen 0,1 und 2,0 g/l, wobei 0,1 - 0,3 g/l besonders bevorzugt ist. Der Gehalt an Gesamtzucker setzt sich zusammen aus dem Gesamtgehalt an Fructose, Glucose und Saccharose, wobei ein Exoenzym der Hefe Saccharose umgehend invertiert, weshalb ggfs. auch eine direkte Saccharosebestimmung entfallen kann. Erfindungsgemäß hat es sich auch gezeigt, dass ein Verhältnis von Glucose- zu Fructosekonzentration vorzugsweise in einem Bereich von 0,1 - 0,5 liegen sollte. Verschiebt sich das Glucose:Fructose-Verhältnis auf etwa > 0,6, so kann dies auf Fehler bei der Vergärung hindeuten. Vorzugsweise wird im erfindungsgemäßen Verfahren dann eine Warnung an den Kellermeister ausgelöst und/qder die Fermentation durch Verringerung der Temperatur verlangsamt oder gestoppt.

Im erfindungsgemäßen Verfahren sollte beim BSA die Abnahme von Malat pro Stunde zwischen 3 mg/l und 50 mg/l betragen, wobei sich diese Werte ausschließlich auf die Durchführung der malolactischen Gärung beziehen. Es ist bevorzugt, den biologischen Säureabbau so ablaufen zu lassen, dass er nach 20 Tagen abgeschlossen ist, da bei längerer Dauer Geschmackseinbußen auftreten. Während der alkoholischen Gärung sollte dieser Wert lediglich als Gärfehlerindikator dienen und sollte zwischen Beginn der Gärung und Gärungsende üblicherweise maximal 1,5 g/l, vorzugsweise max. 1 g/l und insbesonders max. 0,5 g/l schwanken. Werden diese Werte überschritten, dann wird erfindungsgemäß vorzugsweise wie zuvor beschrieben gewarnt und/oder gekühlt. Die Lactatkonzentration ist eine absolute Messgröße mit reinem Informationscharakter. Dieser Wert gibt lediglich Aufschluss darüber ob bereits eine malolactische Gärung erfolgt ist. Sofern auf die Bestimmung des Malatgehaltes verzichtet wird, kann die Steuerung der malolactischen Gärung auch über eine Bestimmung der Lactatänderung erfolgen.

Der Gesamtgehalt an Acetat sollte zweckmäßigerweise einen Maximalwert von 1,8 g/l, vorzugsweise maximal 1,5 g/l, nicht überschreiten. Je nach Art des Getränkes und Geschmackskombination können auch Werte von maximal 0,3 g/l notwendig sein. Dabei sollte der Acetatgehalt so gering als möglich sein. Auch hier wird vorzugsweise beim Überschreiten der Grenzwerte gewarnt und/oder gekühlt. Wird der jeweils gewählte Grenzwert beim erfindungsgemäßen Verfahren nur annähernd erreicht, wird das Getränk ggf. auch nach seiner Fertigstellung stark abgekühlt um so eine weitere Acetatbildung durch unerwünschte Mikroorganismen zu verhindern. Auf diese Weise ist es möglich das fertige Getränk zu filtrieren und diese dafür verantwortlichen Mikroorganismen zu entfernen. Danach kann über das weitere Vorgehen entschieden werden. Die Messung des Gehaltes an Alkohol ist lediglich ein Kontrollstellwert.

Das erfindungsgemäße Verfahren kann auch zur Sedimentationskontrolle eingesetzt werden. Bei der Verarbeitung von zu vergärendem Material, wie beispielsweise Trauben und Obst, werden unerwünschte Trübstoffe in den Most eingetragen, welche durch eine Klärung entfernt werden müssen. Diese Klärung wird üblicherweise mittels einer Sedimentation durchgeführt, wobei sich diese Stoffe am Tankboden absetzen. Es hat sich gezeigt, dass der Klärgrad des Mostes ebenfalls über die spätere Qualität des Produktes entscheidend ist. Dabei wird ein Höchsttrübgehalt von 0,6 % gemessen als Schleudertrübanteil angestrebt. Setzt nun eine ungewollte alkoholische Gärung bereits während des Sedimentationsprozesses unbemerkt ein, so führt dies zum erneuten Aufwirbeln bereits abgesetzter Feststoffe und senkt somit die später resultierende Produktqualität, insbesonders beim Wein. Mittels dem erfindungsgemäßen Verfahren ist es auch möglich den Sedimentationsprozess zu überwachen und bereits in einem frühen Stadium, bei dem die einsetzende Gärung über übliche Temperaturmessungen noch nicht erfasst wird, den jeweiligen Kellermeister zu warnen und die Gärung vorzugsweise durch Kühlung abzubrechen oder zumindest zu verzögern.

Darüber hinaus kann das erfindungsgemäße Verfahren auch zum Überwachen bei der Lagerung von fertigen Weinen eingesetzt werden. Zeigt sich dabei ein ungewollter biologischer Säureabbau (BSA) oder eine Bildung von Essigsäure (Acetat) kann ebenfalls der Kellermeister gewarnt werden bevor es zu einer sensorischen Beeinträchtigung des Produktes kommt. Auf diese Weise kann diese ungewollte Fermentation frühzeitig erkannt werden und sofort Gegenmaßnahmen ergriffen werden.

Die Erfindung betrifft auch eine Vorrichtung zur Durchführung des erfindungsgemäßen Verfahrens, wie sie in den Ansprüchen definiert ist. Eine derartige Vorrichtung umfasst ein Fermentationsgefäß, insbesondere einen Fermentationstank, der mit mindestens einer steuerbaren Kühlvorrichtung ausgestattet ist. Darüber hinaus weist er Einrichtungen auf, mit denen die Temperatur einer im Gefäß vorliegenden Fermentationsbrühe, insbesonders gärende Moste oder Maische, bestimmt werden kann. Diese Temperaturmesseinrichtung kommuniziert mit einer Steuereinrichtung, welche die erhaltenen Messparameter mit gespeicherten Soll- bzw. Sollgrenzwerten vergleicht und so die Kühlleistung der Kühlvorrichtung steuert. Vorzugsweise enthält das Gefäß auch eine Heizvorrichtung zum Erwärmen der Fermentationsbrühe. Vorzugsweise wird die Heizvorrichtung zum Erwärmen der Fermentationsbrühe ebenfalls von der Steuereinheit gesteuert. In einer bevorzugten Ausführungsform enthält die erfindungsgemäße Vorrichtung einen von der Steuereinheit kontrollierten Warner bzw. Alarmgeber mit dem beim Überschreiten der Sollgrenzwerte optisch und/oder akustisch gewarnt wird. Die Erfindung zeichnet sich nun dadurch aus, dass nicht nur die Temperatur der Fermentationsbrühe bestimmt wird, sondern auch die Änderung der Temperatur pro Zeiteinheit. Darüber hinaus enthält die erfindungsgemäße Vorrichtung auch eine Einrichtung zur Ermittlung des Gehaltes bzw. der Änderung des Gehaltes der Fermentationspartner, d.h. der Fermentationssubstrate und -produkte pro Zeit. Eine solche Einrichtung kann eine Analyseeinrichtung auf nasschemischem Wege sein, die ggfs. auch auf Analysechips miniaturisiert sind. Obwohl diese Analysen ohne weiteres von Hand durchführbar sind, ist es erfindungsgemäß bevorzugt, die Analysen mittels einer automatisierten Entnahme und automatisierten A-nalysatoren durchzuführen. Derartige automatisierte Analysatoren sind z. B. aus medizinischen Großlabors bekannt.

Erfindungsgemäß ist es jedoch bevorzugt als eine derartige Einrichtung Sensoren zu verwenden, wie beispielsweise Enzymsensoren und Enzymsonden. Derartige Sensoren sind bekannt. Dabei wird üblicherweise ein Biomaterial auf einen ggf. leitenden oder halbleitenden Träger angeordnet und die Umwandlung der biologischen Funktionen in elektronische Signale beobachtet. Übliche Biomaterialien sind dabei Proteine, Enzyme, Rezeptoren, Antikörper, Antigene, Oligonucleotide, DNA-Fragmente sowie niedermolekulare Moleküle, bei denen Affinitätswechselwirkungen mit anderen Biomaterialien einhergehen. Biosensoren sind beispielsweise auch von Biosensor Applications Sweden AB in Sundbyberg, Schweden kommerziell erhältlich und beispielsweise unter www.biosensor.se erläutert. Weitere Biosensoren, wie beispielsweise Ethanol-Biosensoren, werden vom Zentrum für Angewandte Bioelektrochemische Sensorik ZABS, Marburg/Lahn, Deutschland unter www.zabs-gmbh.de beschrieben. Darüber hinaus ist die Bereitstellung derartiger Sensoren, wie beispielsweise Enzymelektroden, dem Fachmann bekannt und wird beispielsweise von Willner & Katz in "Angew. Chem." 2000, 112, 1230-1269 beschrieben.

Da auch die Größe des Fermentationsgefäßes bzw. Tanks einen großen Einfluss aufgrund der Höhe der Flüssigkeitssäule und des dadurch ausgeübten statischen Drucks auf die Homogenität der Fermentationsbrühe hat, ist es bevorzugt mehrere Proben aus unterschiedlicher Höhe des Tanks zu entnehmen bzw. Sensoren in unterschiedlicher Höhe und an unterschiedlichen Stellen anzuordnen. In manchen Fällen hat es sich als zweckmäßig erwiesen, die so erhaltenen Werte zu mitteln. Vorzugsweise werden Proben aus einer Höhe zwischen einem Fünftel und drei Viertel der Behälterhöhe bzw. der Höhe der Fermentationsbrühe, insbesonders zwischen einem Viertel und der Hälfte der Behälter-/Brühenhöhe, entnommen bzw. Sensoren darin angeordnet. Eine besonders bevorzugte Höhe ist etwa ein Drittel.

Erfindungsgemäß bevorzugte Sensoren leiten die Messparameter, wie z. B. die Konzentration online direkt an eine Steuereinheit weiter.

Die einzelnen Messgrößen bzw. Messparameter sind wie folgt: Temperatur, Temperaturänderung je Zeiteinheit, der Gehalt bzw. die Konzentration des Gesamtzuckers bzw. von Saccharose, Fructose und Glucose sowie deren Abnahme je Zeiteinheit, das Verhältnis von Glucose zu Fructose, der Gehalt bzw. die Konzentration von Malat und Lactat sowie deren Zu- bzw. Abnahme je Zeiteinheit und der Gehalt bzw. die Konzentration von Acetat und Ethanol sowie pH und pH-Änderung jeweils ebenso pro Zeit bzw. Zeiteinheit. Dabei dienen die Werte von Ethanol, Acetat und Lactat sowie des pH-Wertes ebenfalls als Kontrollwerte. Die anderen zuvor genannten Werte bzw. deren Änderung pro Zeit werden verwendet um auf die Gärsteuerung direkt Einfluss zu nehmen. Im Gegensatz zu den Verfahren des Standes der Technik wird erfindungsgemäß dabei Temperatur nicht nur als absolute Größe, sondern auch zusätzlich als Steuerungsgröße für die Temperaturänderung pro Zeit, beispielsweise pro Stunde, verwendet. Es hat sich gezeigt, dass diese Messgröße einen um so stärkeren Einfluss auf die Gärsteuerung hat, je näher der Grenzwert von maximal 5°C, insbesonders maximal 4°C bzw. 3°C je Stunde, erreicht wird.

Soll der Wein vollständig durchgegoren werden, muss üblicherweise die Brühe bei ausklingender Gärung erwärmt werden. Soll ein höherer Restzuckergehalt, d.h. süße Weine, erzeugt werden, wird die Brühe bzw. Most oder Maische durch starke Abkühlung in der Gärung gestoppt und die Hefe dabei inaktiviert. Dabei ist es möglich Weine mit zuvor vom Erzeuger festgelegten Eigenschaften herzustellen. Üblicherweise wird erfindungsgemäß bereits vor Erreichen des Sollwertes mit der Abkühlung begonnen. Je näher am Grenzsollwert, d. h. je später abgekühlt oder erwärmt werden muss, um so größer muss die Kühlleistung oder Heizleistung sein. Da eine hohe Heiz- bzw. Kühlleistung eine starke lokale Unterkühlung/Überhitzung bewirkt, ist sie - wie zuvor beschrieben - unerwünscht. Erfindungsgemäß ist es daher bevorzugt, bereits bei Beginn der Abweichung von Sollwerten die Temperatur zu ändern, um so ein starkes Gegenlenken zu vermeiden. Ausgenommen hierfür ist ein Abkühlen zum Inaktivieren der Mikroorganismen. Dabei wird üblicherweise der Tank mit voller zu Verfügung stehender Kühlleistung auf eine Endtemperatur von ca. 4°C - 5°C abgekühlt und dort gehalten bis der Kellermeister über das weitere Geschehen entschieden hat.

Soll die Bestimmung der erfindungsgemäßen Steuerungsparameter auf nasschemischem Wege erreicht werden, so stehen fertige diagnostische Tests von beispielsweise Roche Diagnostics und Boehringer Mannheim, Deutschland zur Verfügung. Diese stellen kommerziell erhältliche UV-Tests zur Bestimmung von Essigsäure in Lebensmitteln und anderen Probematerialien bereit, wobei das Acetat in Gegenwart des Enzyms Acetyl-CoA-Synthetase mittels Adenosin-5'-triphosphat und Coenzym A zu Acetyl-CoA umgesetzt wird, welches dann mit Oxalacetat in Anwesenheit von Citratsynthase zu Citrat umgesetzt wird.

Die Bestimmung von Milchsäure wird in einem weiteren kommerziell von Roche/Boehringer Mannheim erhältlichen Test ermöglicht, bei dem die Umsetzung von Lactat zum Pyruvat und die dabei gebildete NADH-Menge bestimmt wird. Saccharose und Glucose werden ebenfalls durch einen weiteren kommerziellen Test der zuvor genannten Hersteller mittels Umsetzung mit α-Glucosidase möglich. Auch zur Bestimmung von Glucose und Fructose ist ein Test von Roche Boehringer erhältlich, ebenso ein Ethanol-Test. Weitere Testverfahren sind dem Fachmann bekannt und sind beispielsweise in Nachschlagewerken wie "Methods in Enzymology" beschrieben.

Die Erfindung betrifft auch Nahrungsmittel und Getränke, die mit dem erfindungsgemäßen Verfahren hergestellt werden.

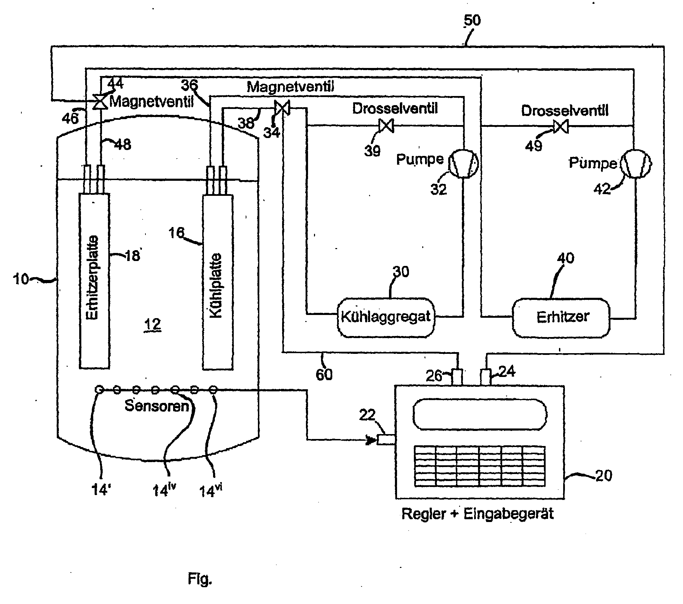

Die Erfindung soll im folgenden anhand der beiliegenden Figur erläutert werden.

Diese Figur zeigt eine erfindungsgemäß bevorzugte Ausführungsform. Darin enthält ein Fermentationstank 10 eine Fermentationsbrühe 12. In diese Fermentationsbrühe 12 taucht ein Kühlelement 16 und ein Heizelement 18 ein. Kühlelement 16 ist mit einem Kühlaggregat 30 über eine ein Kühlmedium enthaltende Zuführleitung 38 und Abführleitung 36 verbunden. Dabei wird das Kühlmedium mittels einer Pumpe 32 im Kreislauf gehalten. Das Heizelement 18 ist mit einem Heizaggregat 40 über eine Zuführleitung 48 und Ableitung 46 verbunden, wobei der Kreislauf des Wärmemediums mittels einer Pumpe 42 aufrecht erhalten wird. Im Gärtank 10 sind Sensoren 14i bis 14vi zur Bestimmung von Temperatur, Saccharose, Fructose und Glucose sowie Malat und ggf. Acetat und Alkohol angeordnet. Dabei stehen die Sensoren 14 in Kommunikation mit einer Regel- und Eingabevorrichtung 20, die über einen Einlass 22 von den Sensoren 14 entsprechende Messwerte erhält. In der Regel- und Eingabevorrichtung 20, welche üblicherweise eine elektronische Datenverarbeitungseinrichtung umfasst, werden die von den Sensoren 14 mitgeteilten Messwerte mit zuvor festgelegten Sollwerten verglichen und bei Abweichungen Steuerventile 34 und 44 entsprechend geregelt, welche das Wärme- oder Kältemedium jeweils zu den Kühl- und Heizelementen leiten. Wird das Steuerventil 34,44 geschlossen, verläuft der Kreislauf im jeweiligen Kühl- oder Heizaggregat über ein zwischengeschaltetes Drosselventil 39 und 49.

Obwohl es erfindungsgemäß bevorzugt ist sowohl ein Heizals auch ein Kühlelement im Fermentationstank anzuordnen, ist es erfindungsgemäß auch möglich auf ein Heizelement vollständig zu verzichten, da der Fermentationsprozess exotherm verläuft, d.h. Wärme freisetzt, welche zum Erhitzen der Fermentationsbrühe verwendet werden kann. Die Steuerung erfolgt in diesem Fall allein durch das Kühlelement. Mit dem erfindungsgemäßen Verfahren ist es möglich eine je nach Ernte jahrgangsabhängige Fermentation bzw. Gärung zu optimieren, wobei der Kellermeister den Charakter des Endweines nach seinen persönlichen Erfahrungen optimieren kann.

## Patentansprüche

1. Verfahren zur Herstellung von alkoholischen und/oder milchsäurehaltigen Nahrungsmitteln und/oder Getränken mittels Mikroorganismen und/oder daraus gewonnenen Fermenten in einer wässrigen Fermentationsbrühe durch Erfassen und Steuern der Temperatur der Brühe und von in der Brühe vorliegenden Fermentationsparametern, **dadurch gekennzeichnet, dass** mindestens zwei Parameter erfasst werden, die ausgewählt sind aus Temperaturänderung pro Zeit, Abnahme der Gesamtzuckerkonzentration pro Zeit, Verhältnis von Glucose- zu Fructosekonzentration, Abnahme von Malatkonzentration pro Zeit, Zunahme von Lactatkonzentration pro Zeit und dass
beim Überschreiten von Sollgrenzwerten von mindestens einem dieser Parameter die Temperatur der Fermentationsbrühe durch Erwärmen oder Kühlen soweit geändert wird, bis der oder die Parameter wieder innerhalb von Sollgrenzwerten liegen oder eine unerwünschte Stoffwechselreaktion minimiert wird.

2. Verfahren nach Anspruch 1, **dadurch gekennzeichnet, dass** die Konzentration der Fermentationsparameter in der Fermentationsbrühe bestimmt wird.

3. Verfahren nach einem der vorhergehenden Ansprüche, **dadurch gekennzeichnet, dass** die Bestimmung der Fermentationsparameter mittels Biosensoren erfolgt.

4. Verfahren nach einem der vorhergehenden Ansprüche, **dadurch gekennzeichnet, dass** die Bestimmung mittels Enzymelektroden erfolgt.

5. Verfahren nach einem der vorhergehenden Ansprüche, **dadurch gekennzeichnet, dass** eine Abnahme des Gesamtzukkergehaltes von 0,05 g/l pro Stunde bis 2,2 g/l pro Stunde eingestellt wird.

6. Verfahren nach einem der vorhergehenden Ansprüche, **dadurch gekennzeichnet, dass** die Menge an Fructose, Glucose und Saccharose bestimmt und daraus der Gesamtzuckergehalt ermittelt wird.

7. Verfahren nach einem der vorhergehenden Ansprüche, **dadurch gekennzeichnet, dass** bei einem Verhältnis von Glucose zu Fructose von > 0,6 eine Warnung erfolgt

8. Verfahren nach einem der vorhergehenden Ansprüche, **dadurch gekennzeichnet, dass** bei der malolactischen Gärung die Abnahme der Malatkonzentration von 3 mg/l und Stunde bis 50 mg/l und Stunde eingestellt wird.

9. Verfahren nach einem der vorhergehenden Ansprüche, **dadurch gekennzeichnet, dass** bei einer Konzentration von Acetat über maximal 1,5 g/l eine Warnung erfolgt.

10. Verfahren nach einem der vorhergehenden Ansprüche, **dadurch gekennzeichnet, dass** die Fermentation bei einer Temperatur zwischen 3 - 35°C durchgeführt wird.

11. Vorrichtung zur Herstellung von alkoholischen und/oder milchsäurehaltigen Nahrungsmitteln und/oder Getränken mittels Mikroorganismen und/oder daraus gewonnenen Fermenten in einer wässrigen Fermentationsbrühe umfassend ein Fermentationsgefäß mit einer Temperaturmesseinrichtung, mindestens einer Einrichtung zum Ermitteln von Fermentationsparametern sowie mindestens einer steuerbaren Kühlvorrichtung und eine Steuereinrichtung mindestens zum Steuern der Kühlvorrichtung, **dadurch gekennzeichnet, dass** die Temperaturmesseinrichtung die Temperaturänderung pro Zeit bestimmt und die Steuereinrichtung die so erhaltenen Werte mit gespeicherten Sollgrenzwerten vergleicht und die Kühlvorrichtung steuert und die Einrichtung zur Ermittlung der Fermentationsparameter die Abnahme der Gesamtzuckerkonzentration pro Zeit, Verhältnis von Glucose- zu Fructosekonzentration, Abnahme von Malatkonzentration pro Zeit und/oder Zunahme von Lactatkonzentration pro Zeit ermittelt und an die Steuereinrichtung leitet.

## Claims

1. Method for manufacturing foodstuffs and/or beverages which are alcoholic and/or contain lactic acid, said method using micro organisms and/or enzymes obtained therefrom in an aqueous fermentation broth by recording and controlling the temperature of the broth and from fermentation parameters present in the broth, **characterized in that** at least two parameters are recorded, selected from temperature change with time, reduction in the total sugar concentration with time, ratio of glucose concentration to fructose concentration, reduction in malate concentration with time, increase in lactate concentration with time, and that where required limit values of at least one of said parameters are exceeded, the temperature of the fermentation broth is changed by heating or cooling until the parameter or parameters is or are once again within the required limits or an unwanted metabolism reaction is minimised.

2. Method according to claim 1, **characterized in that** the concentration of the fermentation parameters in the fermentation broth is determined.

3. Method according to one of the preceding claims, **characterized in that** the determining of the fermentation parameters is effected by means of biosensors.

4. Method according to one of the preceding claims, **characterized in that** the determining is effected by means of enzyme electrodes.

5. Method according to one of the preceding claims, **characterized in that** a reduction in the total sugar content is set between 0.05 g/l per hour and 2.2 g/l per hour.

6. Method according to one of the preceding claims, **characterized in that** the quantity of fructose, glucose and sucrose is defined and the total sugar content is determined therefrom.

7. Method according to one of the preceding claims, **characterized in that** at a ratio of glucose to fructose of > 0.6, a warning is given.

8. Method according to one of the preceding claims, **characterized in that** with malolactic fermentation, the reduction in the malate concentration is set between 3 mg/l and hour and 50 mg/l and hour.

9. Method according to one of the preceding claims, **characterized in that** where there is a concentration of acetate in excess of a maximum or 1.5 g/l, a warning is given.

10. Method according to one of the preceding claims, **characterized in that** the fermentation is carried out at a temperature between 3 - 35° C.

11. Device for manufacturing foodstuffs and/or beverages which are alcoholic and/or contain lactic acid, said device using micro organisms and/or enzymes obtained therefrom in an aqueous fermentation broth and comprising a fermentation vessel with a temperature measuring device, at least one device for determining fermentation parameters and at least one controllable cooling device and a control device at least for controlling the cooling device, **characterized in that** the temperature measuring device determines the temperature change with time and the control device compares the values obtained in this manner with stored required limit values and controls the cooling device and the device for determining the fermentation parameters determines the reduction in the total sugar concentration with time, the ratio of glucose concentration to fructose concentration, the reduction in malate concentration with time and/or the increase in lactate concentration with time, and conducts such to the control device.

## Revendications

1. Procédé de fabrication de produits alimentaires et/ou de boissons alcoolisés et/ou contenant de l'acide lactique à l'aide de micro-organismes et/ou de ferments qui en sont extraits dans un bouillon de fermentation aqueux en saisissant et en commandant la température du bouillon et les paramètres de fermentation présents dans le bouillon, **caractérisé en ce qu'**au moins deux paramètres sont saisis, lesquels sont sélectionnés parmi la variation de température par unité de temps, la diminution de la concentration totale en sucre par unité de temps, le rapport entre la concentration de glucose et la concentration de fructose, la diminution de la concentration de malate par unité de temps, l'augmentation de la concentration de lactate par unité de temps et **caractérisé en ce que,**
lorsqu'au moins un des ces paramètres dépasse des valeurs seuil théoriques, la température du bouillon de fermentation est modifiée par échauffement ou refroidissement jusqu'à ce que le ou les paramètres se situent à nouveau dans les valeurs seuil théoriques ou jusqu'à ce qu'une réaction métabolique indésirable soit minimisée.

2. Procédé selon la revendication 1, **caractérisé en ce que** la concentration des paramètres de fermentation est déterminée dans le bouillon de fermentation.

3. Procédé selon l'une quelconque des revendications précédentes, **caractérisé en ce que** la détermination des paramètres de fermentation est effectuée à l'aide de biocapteurs.

4. Procédé selon l'une quelconque des revendications précédentes, **caractérisé en ce que** la détermination est effectuée à l'aide d'électrodes enzymatiques.

5. Procédé selon l'une quelconque des revendications précédentes, **caractérisé en ce que** l'on règle une diminution de la teneur totale en sucre de 0,05 g/l par heure jusqu'à 2,2 g/l par heure.

6. Procédé selon l'une quelconque des revendications précédentes, **caractérisé en ce que** la quantité de fructose, de glucose et de saccharose est déterminée et permet d'établir la teneur en sucre totale.

7. Procédé selon l'une quelconque des revendications précédentes, **caractérisé en ce qu'**un avertissement a lieu dans le cas d'un rapport entre le glucose et le fructose supérieur à 0,6.

8. Procédé selon l'une quelconque des revendications précédentes, **caractérisé en ce que,** lors de la fermentation malolactique, on règle la diminution de la concentration en malate de 3 mg/l et par heure jusqu'à 50 mg/l et par heure.

9. Procédé selon l'une quelconque des revendications précédentes, **caractérisé en ce qu'**un avertissement a lieu pour une concentration d'acétate au-dessus d'un maximum de 1,5 g/l.

10. Procédé selon l'une quelconque des revendications précédentes, **caractérisé en ce que** la fermentation est effectuée à une température située entre 3 et 35 °C.

11. Dispositif de fabrication de produits alimentaires et/ou de boissons alcoolisés et/ou contenant de l'acide lactique à l'aide de micro-organismes et/ou de ferments qui en sont extraits dans un bouillon de fermentation aqueux, comportant un récipient de fermentation doté d'un dispositif de mesure de température, d'au moins un dispositif servant à déterminer les paramètres de fermentation ainsi qu'au moins d'un dispositif de refroidissement pouvant être commandé et comportant un dispositif de commande servant à commander au moins le dispositif de refroidissement, **caractérisé en ce que** le dispositif de mesure de température détermine la variation de température par unité de temps, **en ce que** le dispositif de commande compare les valeurs ainsi obtenues avec des valeurs seuil théoriques enregistrées et commande le dispositif de refroidissement, et **en ce que** le dispositif permettant de déterminer les paramètres de fermentation détermine la diminution de la concentration totale en sucre par unité de temps, le rapport entre la concentration de glucose et de fructose, la diminution de la concentration de malate par unité de temps et/ou l'augmentation de la concentration de lactate par unité de temps, et transmet ces informations au dispositif de commande.
